# EUROPEAN PATENT APPLICATION

(11) **EP 2 441 451 A1**
(43) Date of publication of application: **18.04.2012**
(21) Application number: 10786158.5
(22) Date of filing: 08.06.2010
(51) Int. Cl.: A61K 31/05, A23K 1/16, A61K 36/00, A61P 33/02

(54) **COCCIDIOSIS CONTROL AGENT AND FEED CONTAINING SAME**

(30) Priority: 08.06.2009 JP 2009137565
(71) Applicant: Idemitsu Kosan Co., Ltd., Chiyoda-ku Tokyo 100-8321 (JP)
(72) Inventor: MOCHIZUKI, Masami, Sodegaura-shi Chiba 299-0293 (JP); NAGASHIMA, Kyo, Sodegaura-shi Chiba 299-0293 (JP); HIKITA, Chie, Tokyo 107-0061 (JP)
(74) Representative: Berryman, Natalia Grace
(86) International application number: PCT/JP2010/059691
(87) International publication number: WO 2010/143627

(57) **Abstract**

An object of the present invention is to provide: a coccidiosis controlling agent which is excellent in safety, is free of problems such as side-effects, does not cause effect reduction or the like due to the acquisition of resistance to a medicament, and exhibits a high preventive effect and a high treatment effect against coccidiosis; a feed for an animal containing the anticoccidial composition; and a method for raising an animal using the coccidiosis controlling agent and the feed. In order to solve the above-mentioned problem, provided is a coccidiosis controlling agent for an animal, containing heated cashew nut shell liquid and/or cardanol.

## Description

### Technical Field

The present invention relates to a coccidiosis controlling agent for an animal, an additive for feed, and a feed each containing heated cashew nut shell liquid and/or cardanol, and a method for raising an animal using those.

### Background Art

Coccidiosis of an animal is a parasitic protozoan infection, which is infected through oral ingestion of oocysts (capsule of aggregate of sporozoite). The infection causes pathologies such as intestinal tract lesion, diarrhea, anorexia, extinction, and weight loss, and there may also be a case where the animal dies. Further, when an animal is infected with coccidiosis, intestinal microbiological flora collapses, and in addition, diarrhea particularly becomes severe due to the mixed infection with pathogenic bacteria. Thus, the animal exhausts itself, immunocompetence thereof is lowered, and hence the animal is easily infected with necrotic enteritis (bacterial infection in intestinal tract inner layer, which brings about necrosis of various regions of intestinal tract inner layer in digestive tract).
It is known that coccidiosis of a ruminant is caused by *Eimeria bovis, E. zuernii, E. auburnensis, E. ellipsoidalis, E. arloingi, E. ovina,* and the like.
On the other hand, it is known that coccidiosis of a chicken is caused by *Eimeria tenella, E. acervulina, E. necatrix, E. brunetti, E. maxima, E. mivati, E. mitis, E. precox, E. hagani,* or the like, and coccidiosis of a turkey is caused by *E. meleagrimitis, E. adenoides, E. gallopovonis,* and the like.
As is clear from the above, the kinds of parasitic protozoas belonging to the genus *Eimeria* on ruminants are different from those on chickens and turkeys, and the parasitism of the protozoa belonging to the genus *Eimeria* is remarkably host specific.

Conventionally, in preventing or treating the coccidiosis of an animal, there have been mainly used antibiotics (polyether-based antibiotics such as salinomycin), chemotherapeutic agents comprised of synthetic antibacterial agents (sulfa drug and the like), and biological preparations such as vaccine. However, the use of the antibiotics or the chemotherapeutic agents has problems of development of side effects and effect reduction or the like due to the acquisition of resistance to the medicament. Further, vaccine could be used only for the prevention, and was not able to be used for treating the coccidiosis. In addition, when meat, eggs, and the like of the animals to which those medicaments have been administered are used for human consumption, there is a problem that the medicaments remaining in the animal body were transferred to the human body. Therefore, there have been strict restrictions on the amount used and period for administration of the medicaments.
Consequently, there has been demanded a coccidiosis preventing or alleviating agent which is free of the above-mentioned problems caused by the antibiotics, synthetic antibacterial agents, vaccine, and the like, is highly safe, and is excellent in anticoccidial effect. As a response, a series of coccidiosis alleviating agents (Patent Documents 1 to 3) each containing, as an active ingredient, cashew nut shell liquid and/or anacardic acids, which are main components of the cashew nut shell liquid have been proposed. However, effects thereof have not been sufficient, and further improvement in the effects has been demanded.

### Prior Art Documents

### Patent Documents

Patent Document 1: JP 2003-238400 A
Patent Document 2: JP 2001-151675 A
Patent Document 3: JP 08-231410 A

### Summary of the Invention

An object of the present invention is to provide: a coccidiosis controlling agent which is excellent in safety, is free of problems such as side-effects, does not cause effect reduction or the like due to the acquisition of resistance to a medicament, and exhibits a high preventive effect and a high treatment effect against coccidiosis; a feed for an animal containing the coccidiosis controlling agent; and a method for raising an animal using the coccidiosis controlling agent and the feed.

The inventors of the present invention have conducted intensive studies in order to solve the above problems, and as a result, the inventors have found that, when heated cashew nut shell liquid and/or cardanol is used, additional effects in the prevention and the treatment against coccidiosis of an animal are observed as compared with non-heated cashew nut shell liquid.
Thus, the inventors of the present invention have completed the present invention.

That is, the present invention is as follows:
(1) A coccidiosis controlling agent for an animal comprising heated cashew nut shell liquid and/or cardanol.
(2) An additive for feed comprising the coccidiosis controlling agent according to (1).
(3) A feed comprising the coccidiosis controlling agent according to (1) or the additive for feed according to (2).
(4) A method for raising an animal comprising feeding the animal with the feed according to (3).
(5) Heated cashew nut shell liquid and/or cardanol for use in producing a coccidiosis controlling agent for an animal.
(6) A method for controlling coccidiosis of an animal, comprising administering heated cashew nut shell liquid and/or cardanol to the animal which requires controlling coccidiosis.
(7) Heated cashew nut shell liquid and/or cardanol which is used for controlling coccidiosis of an animal.

According to the coccidiosis controlling agent of the present invention, and the additive for feed and the feed of the present invention each containing the coccidiosis controlling agent, the coccidiosis of an animal can be alleviated. Further, according to the coccidiosis controlling agent of the present invention, and the additive for feed and the feed of the present invention each containing the coccidiosis controlling agent, improvements in the body weight gain and the growth of an animal can be expected.

### Mode for carrying out the Invention

The coccidiosis controlling agent of the present invention contains heated cashew nut shell liquid and/or cardanol.

The cashew nut shell liquid is an oily liquid contained in the shell of the seed of a cashew nut tree (*Anacardium occidentale L*.). The cashew nut shell liquid contains, as components thereof, anacardic acid, cardanol, and cardol. In general, anacardic acid is converted into cardanol by a heating treatment.
Non-heated cashew nut shell liquid extracted by compressing the shell of a cashew nut contains 55 to 80 mass% anacardic acid, 5 to 20 mass% cardanol, and 5 to 30 mass% cardol as described in J. Agric. Food Chem. 2001, 49, 2548-2551.
Heated cashew nut shell liquid obtained by heat-treating non-heated cashew nut shell liquid at 130°C or higher contains 0 to 10 mass% anacardic acid, 55 to 80 mass% cardanol, and 5 to 30 mass% cardol, because anacardic acid which is a major component of non-heated cashew nut shell liquid is converted into cardanol by decarboxylation.

The cashew nut shell liquid can be obtained as a vegetable oil extracted by compressing the shell of a cashew nut. Further, the cashew nut shell liquid can also be obtained by extracting, e.g., solvent-extracting a cashew nut shell. In addition, the cashew nut shell liquid can be obtained according to a method described in JP 08-231410 A, e.g., by a solvent extraction method.
The cashew nut shell liquid may also be a liquid obtained by pulverizing/crushing the shell of a cashew nut. Further, a commercially available cashew nut shell liquid product may also be used.
The heated cashew nut shell liquid of the present invention can be obtained by heating non-heated cashewnut shell liquid obtained as above to 70°C or higher, preferably 130°C or higher.
The heated cashew nut shell liquid of the present invention is preferably obtained by performing compression and extraction of the shell of a cashew nut and heat-treating the resultant product (non-heated cashew nut shell liquid) at 130°C.

The heated cashew nut shell liquid of the present invention may be obtained by, for example, dry distillation of the shell of a cashew nut. In addition, the heated cashew nut shell liquid may be obtained by the method described in JP 08-231410 A. Specifically, the heated cashew nut shell liquid may be obtained by adding the shell of a cashew nut to heated cashew nut shell liquid heated to 200 to 240° C and extracting heated cashew nut shell liquid. Moreover, the heated cashew nut shell liquid may be a commercially available product which has been already heated.

The content of the heated cashew nut shell liquid in the coccidiosis controlling agent of the present invention is preferably 10 mass% to 100 mass%, more preferably 15 mass% to 100 mass%, and still more preferably 20 mass% to 100 mass%. When the content is 10 mass% or more, the anticoccidial effect can be exhibited with a predetermined amount of the controlling agent.
The content of the coccidiosis controlling agent in the additive for feed of the present invention is preferably 10 mass% to 100 mass%, more preferably 15 mass% to 100 mass%, still more preferably 20 mass% to 100 mass%. When the content is 10 mass% or more, the anticoccidial effect can be exhibited with a predetermined amount of the additive for feed.
The content of the additive for feed in the feed of the present invention is preferably 0.01 mass% to 20 mass%, more preferably 0.05 mass% to 20 mass%, still more preferably 0.1 mass% to 20 mass%. When the content is 0.01 mass% or more, the anticoccidial effect can be exhibited with a predetermined amount of the controlling agent.

When the coccidiosis controlling agent of the present invention is contained in a feed and used, the content of the heated cashew nut shell liquid in the feed may be set to preferably 0.02 mass% to 4.0 mass%, more preferably 0.04 mass% to 2.0 mass%, and still more preferably 0.06 mass% to 1.0 mass%. It is preferred that the content be 0.02 mass% or more, because the anticoccidial effect can be exhibited with a predetermined amount of the feed, and that the content be 4.0 mass% or less, because the cashew nut shell liquid does not have an influence on the feed composition.
For the heated cashew nut shell liquid used in the present invention, the shell of a cashew nut containing oil may be used as it is, or may be pulverized/crushed, and further subjected to a heating treatment before or after the pulverizing/crushing, and product thereof may be used, and the cashew nut shell liquid may be contained so that the contents of the cashew nut shell liquid in the coccidiosis controlling agent, the additive for feed, and the feed are within the above ranges in terms of the cashew nut shell liquid (CNSL) (25 to 30 mass% of CNSL is contained in the cashew nut shell).

Examples of the cardanol used in the present invention include natural cardanol, synthetic cardanol, and derivatives thereof. The cardanol used in the present invention can be obtained by decarboxylation of anacardic acid which is a major component of cashew nut shell liquid.
Note that the mass ratio of anacardic acid to cardanol in the heated cashew nut shell liquid of the present invention is preferably 0:100 to 20:80.

Examples of the target animals for the coccidiosis controlling agent of the present invention preferably include livestock or pets such as cows, pigs, and chickens, but are not limited thereto. Note that, in the present invention, the term "controlling" includes prevention and treatment.

Examples of coccidia which cause chicken coccidiosis to be controlled by the coccidiosis controlling agent of the present invention include *Eimeria tenella, E. acervulina, E. necatrix, E. brunetti, E. maxima, E. mivati, E. mitis, E. precox,* and *E*. *hagani.* Examples of coccidia which cause turkey coccidiosis include *E*. *meleagrimitis, E. adenoides,* and *E. gallopovonis.*
Examples of coccidia which cause cow coccidiosis include *Eimeria bovis, E. zuernii, E. auburnesis, E. ellipsoidalis, E. arloingi,* and *E. ovina.*

The formulation of the coccidiosis controlling agent of the present invention is not particularly limited, and the agent may be in an arbitrary form such as powder, liquid, solid, a tablet, a capsule, or emulsion. The coccidiosis controlling agent of the present invention can be produced by mixing heated cashew nut shell liquid and/or cardanol, and if necessary, an arbitrary component, and forming the mixture into a preparation. Note that, depending on the form of the formulation, the pulverized/crushed product of the heated cashew nut shell or the cashew nut shell as it is without being subjected to any treatment other than a heating treatment is mixed with another arbitrary component, and the mixture can be used as the coccidiosis controlling agent of the present invention. In addition, without being mixed with another arbitrary component, the heated pulverized/crushed product as it is or the heated cashew nut shell as it is may be used as the additive for feed, or further, as the feed.

An additive for feed may be produced by appropriately mixing heated cashew nut shell liquid and/or cardanol, and diatomaceous earth, bentonite, montmorillonite, zeolite, pearlite, acid clay, activated clay, or silicic acid in the coccidiosis controlling agent of the present invention.

The additive for feed of the present invention may be used as a feed by mixing the additive for feed with another feed component used in pet foods and supplements for pets (hereinafter referred to as feed). The kind of the feed and the components other than the cashew nut shell liquid are not particularly limited. The feed is preferably a feed for livestock or pets.

The feed of the present invention can be produced by adding an additive for feed as it is to a feed component and mixing the resultant. On this occasion, when a powdery or solid additive for feed is used, the form of the additive for feed may be modified into a liquid form or a gel form for the purpose of facilitating the mixing process. In this case, the following may be used as a liquid carrier: water; a vegetable oil such as soybean oil, rapeseed oil, or corn oil; liquid animal oil; or a water-soluble polymer compound such as polyvinyl alcohol, polyvinylpyrrolidone, or polyacrylic acid. Further, in order to keep the uniformity of the cashew nut shell liquid in the feed, the feed preferably contains alginic acid, sodium alginate, a xanthan gum, casein sodium, an arabic rubber, a guar gum, or a water-soluble polysaccharide such as tamarind seed polysaccharide.

The feed of the present invention may contain sugars (such as lactose and trehalose), maize, milo, bran, rice bran, defatted bran, dried bran, barley flake, corn flake, soybean meal, corn flour, rice flour, soybean flour, and the like. Their concentrations in the feed are preferably 1 to 90 mass%, more preferably 5 to 75 mass%, and still more preferably 10 to 50 mass%.

The feed of the present invention may further contain an arbitrary component such as a component which is effective for the growth promotion of livestock or pets, a nutritional supplement component, or a component for enhancing the preservation stability. Examples of the arbitrary component include: probiotics such as *Enterococcus, Bacillus, Bifidus,* and *Lactobacillus;* enzymes such as amylase and lipase; vitamins such as choline chloride, inositol, and folic acid; minerals such as potassium chloride, iron citrate, and phosphates; amino acids such as DL-alanine, DL-methionine, L-lysine hydrochloride; organic acids such as fumaric acid, butyric acid, lactic acid, acetic acid, and their salts; antioxidants such as ethoxyquin and dibutylhydroxytoluene; fungicides such as propionic acid and sodium propionate; binders such as CMC, casein sodium, and sodium polyacrylate; emulsifiers such as glycerin fatty acid ester and sorbitan fatty acid ester; pigments such as astaxanthin and canthaxanthin; and flavoring agents such as various esters, ethers, and ketones.

The feed of the present invention is suitable for breeding livestock or pets such as cows, pigs, and chickens. The amount of feed ingested by an animal may be appropriately adjusted depending on the animal's species, body weight, age, sex, health condition, feed component, etc. In this case, the amount of heated cashew nut shell liquid and/or cardanol contained in the feed is preferably 0.005 to 500 g per animal per day, more preferably 0.05 to 100 g per animal per day, and still more preferably 0.5 to 50 g per animal per day.
Any method usually used may be adopted as a method of feeding animals and a method of raising animals depending on the species of animals.

### Examples

Hereinafter, Examples of the present invention are described, but the present invention is not limited to those Examples.

### 1. Production Example

Cashew nut shells (500 kg) were purchased from Cashew Trading Co., Ltd., and the shells were compressed, thereby producing 158 kg of cashew nut shell liquid (non-heated CNSL). Meanwhile, heat-treated cashew nut shell liquid (heated CNSL) (the coccidiosis controlling agent of the present invention) obtained by a heat-treatment to convert anacardic acid into cardanol was purchased from Cashew Trading Co., Ltd.
The composition of CNSL was measured by the following method. That is, HPLC (Waters 600, Nihon Waters K.K.), a detector (Waters 490E, Nihon Waters K.K.), a printer (Chromatopak C-R6A, Shimadzu Corporation), and a column (SUPELCOSIL LC18, SUPELCO, Inc.) were used. A solvent including acetonitrile: water: acetic acid=80:20:1 (volume ratio) was used, and the flow rate was adjusted to 2 ml/min. Detection was performed at an absorbance of 280 nm.
The non-heated cashew nut shell liquid was found to contain 61.8 mass% anacardic acid, 8.2 mass% cardanol, and 19.9 mass% cardol, while the heated cashew nut shell liquid was found to contain 0.0 mass% anacardic acid, 71.4 mass% cardanol, and 14.4 mass% cardol.

### 2. Cow coccidium-control test

Diarrheal feces of cows that had been naturally infected with *Eimeria zuernii* were collected, and oocysts were suspended in 5 ml of physiological saline at about 1500 oocysts/ml. A group obtained by adding only physiological saline to a vial container was defined as a control group, and groups obtained by separately adding non-heated CNSL at 0.5 mass%, 0.05 mass%, and 0.005 mass% to vial containers were defined as Comparative Examples (Comparative Examples 1, 2, and 3). Groups obtained by separately adding heated CNSL at 0.5 mass%, 0.05 mass%, and 0.005 mass% to vial containers were defined as Examples (Examples 1, 2, and 3). The vial containers were left standing still at 37°C to conduct timely observations. 7 days later, a stereomicroscope was used to determine the numbers of oocysts and to observe conditions of deformed cell walls and dissolved cell walls of oocysts.
The results are shown in Table 1.

**[Table 1]**

| | Concentration (mass%) | Number of viable oocysts | | Reduction rate (%) | Rate of dissolved and deformed oocysts¹⁾ (%) |
|---|---|---|---|---|---|
| | | Day 0 | Day 7 | | |
| Comparative Example 1 | 0.5 | 1500 | 1200 | 20 | 50 |
| Comparative Example 2 | 0.05 | 1500 | 1400 | 7 | 17 |
| Comparative Example 3 | 0.005 | 1500 | 1500 | 0 | 0 |
| Example 1 | 0.5 | 1500 | 100 | 93 | 100 |
| Example 2 | 0.05 | 1500 | 800 | 47 | 25 |
| Example 3 | 0.005 | 1500 | 1500 | 0 | 0 |
| Control Example | 0.0 | 1500 | 1500 | 0 | 0 |

| | | | | | |
|---|---|---|---|---|---|
| ¹⁾ Rate of oocysts that remained on day 7 | | | | | |

As shown in Examples 1 to 3, there were observed reductions in the numbers of oocysts of *Eimeria zuernii* and the dissolving and deformation in the remained oocysts with one-tenth concentration of the coccidiosis controlling agent of the present invention (heated CNSL) compared with Comparative Examples 1 to 3 (non-heated CNSL).
As described above, the effect of the coccidiosis controlling agent of the present invention can be exerted by adding 0.05 mass% or more of the heated cashew nut shell liquid to physiological saline containing oocysts of cow coccidium at 1500 oocysts/ml.

### 3. Chicken coccidium-control test

Diarrheal feces of chickens that had been naturally infected with *Eimeria tenella* were collected, and oocysts were suspended in 5 ml of physiological saline at about 4000 oocysts/ml. A group obtained by adding only physiological saline to a vial container was defined as a control group, and groups obtained by separately adding non-heated CNSL at 0.5 mass%, 0.05 mass%, and 0.005 mass% to vial containers were defined as Comparative Examples (Comparative Examples 4, 5, and 6). Groups obtained by separately adding heated CNSL at 0.5 mass%, 0.05 mass%, and 0.005 mass% to vial containers were defined as Examples (Examples 4, 5, and 6). The vial containers were left standing still at 37°C to conduct timely observations. 7 days later, a stereomicroscope was used to determine the numbers of oocysts and to observe conditions of deformed cell walls and dissolved cell walls of oocysts.
The results are shown in Table 2.

**[Table 2]**

| | Concentration (mass%) | Number of viable oocysts | | Reduction rate | Rate of dissolved and deformed oocysts¹⁾ (%) |
|---|---|---|---|---|---|
| | | Day 0 | Day 7 | (%) | |
| Comparative Example 4 | 0.5 | 4000 | 3200 | 20 | 78 |
| Comparative Example 5 | 0.05 | 4000 | 3600 | 10 | 9 |
| Comparative Example 6 | 0.005 | 4000 | 4000 | 0 | 0 |
| Example 4 | 0.5 | 4000 | 400 | 90 | 100 |
| Example 5 | 0.05 | 4000 | 2100 | 48 | 10 |
| Example 6 | 0.005 | 4000 | 4000 | 0 | 0 |
| Control Example | 0.0 | 4000 | 4000 | 0 | 0 |

| | | | | | |
|---|---|---|---|---|---|
| ¹⁾ Rate of oocysts that remained on day 7 | | | | | |

As shown in Examples 4 to 6, there were observed reductions in the numbers of oocysts of *Eimeria tenella* and the dissolving and deformation in the remained oocysts with one-tenth concentration of the coccidiosis controlling agent of the present invention (heated CNSL) compared with Comparative Examples 4 to 6 (non-heated CNSL).
As described above, the effect of the coccidiosis controlling agent of the present invention can be exerted by adding 0.05 mass% or more of the heated cashew nut shell liquid to physiological saline containing oocysts of chicken coccidium at 4000 oocysts/ml.

### 4. Preparation example of additive for feed

Additives for feed having the following compositions were prepared.

| (1) Production Example 1 | |
|---|---|
| Heated cashew nut shell liquid | 35 mass% |
| Diatomaceous earth (Product of Wako Pure Chemical Industries, Ltd.) | 65 mass% |

| (2) Production Example 2 | |
|---|---|
| Heated cashew nut shell liquid Acrid clay (trade name: MIZUKA-ACE #20, product of MIZUSAWA INDUSTRIAL | 20 mass% |
| CHEMICALS, LTD.) | 80 mass% |

| (3) Production Example 3 | |
|---|---|
| Heated cashew nut shell liquid | 35 mass% |
| Activated clay (trade name: Galleon Earth V2, product of MIZUSAWA INDUSTRIAL CHEMICALS, LTD.) | 65 mass% |

| (4) Production Example 4 | |
|---|---|
| Heated cashew nut shell liquid | 20 mass% |
| Zeolite (product of TIME CHEMICAL Corporation.) | 80 mass% |

| (5) Production Example 5 | |
|---|---|
| Heated cashew nut shell liquid | 67 mass% |
| Silica (sipernat 22, product of Evonik Degussa Japan CO., Ltd.) | 33 mass% |

### 5. Production Examples of feeds

Feeds for chickens or cows each having a composition described below.

### (1) Production Examples of feed for chickens

### (i) Production Example 1: Production Example of feed for broiler chickens

| | |
|---|---|
| Corn | 57.41 mass% |
| EX soybean (soybean extract) | 2.00 mass% |
| Soybean meal | 26.50 mass% |
| Corn gluten meal | 5.00 mass% |
| Soybean oil | 4.90 mass% |
| Calcium phosphate | 1.89 mass% |
| Calcium carbonate | 0.68 mass% |
| Dietary salt | 0.20 mass% |
| Lysine | 0.30 mass% |
| Choline chloride | 0.02 mass% |
| Vitamin premix* | 0.40 mass% |
| Methionine | 0.20 mass% |
| Heated CNSL | 0.50 mass% |

| | |
|---|---|
| *Vitamin premix: mixed product of vitamin A, vitamin D3, vitamin E, vitamin B1, vitamin B2, vitamin B6, nicotinic acid, pantothenic acid, biotin, folic acid, and vitamin B12 | |

(ii) Production Example 2: Production Example of feed for broiler chickens

| | |
|---|---|
| Standard feed for a broiler-fattening earlier stage (SDB No. 1) (product of Nippon Formula Feed Mfg Co., Ltd.) | 99.50 mass% |
| Heated CNSL | 0.50 mass% |

(iii) Production Example 3: Production Example of feed for broiler chickens

| | |
|---|---|
| Standard feed for a broiler-fattening later stage (SDB No. 2) (product of Nippon Formula Feed Mfg Co., Ltd.) | 99.50 mass% |
| Heated CNSL | 0.50 mass% |

(iv) Production Example 4: Production Example of feed for layer chickens

| | |
|---|---|
| Standard feed for young chicks (SDL No. 1) (product of Nippon Formula Feed Mfg Co., Ltd.) | 99.50 mass% |
| Heated CNSL | 0.50 mass% |

(v) Production Example 5: Production Example of feed for layer chickens

| | |
|---|---|
| Standard feed for old chicks (SDL No. 2) (product of Nippon Formula Feed Mfg Co., Ltd.) | 99.50 mass% |
| Heated CNSL | 0.50 mass% |

(vi) Production Example 6: Production Example of feed for layer chickens

| | |
|---|---|
| Standard feed for pullets (SDL No. 3) (product of Nippon Formula Feed Mfg Co., Ltd.) | 99.50 mass% |
| Heated CNSL | 0.50 mass% |

(vii) Production Example 7: Production Example of feed for layer chickens

| | |
|---|---|
| Standard feed for adult chickens (SDL No. 4) (product of Nippon Formula Feed Mfg Co., Ltd.) | 99.50 mass% |
| Heated CNSL | 0.50 mass% |

### (2) Production Examples of feed for cows

(viii) Production Example 8: Production Example of feed for calves

| | |
|---|---|
| Coarse-grained corn | 40.89 mass% |
| Granular corncob | 34.50 mass% |
| Soybean meal | 8.10 mass% |
| Alfalfa meal | 4.00 mass% |
| Molasses | 10.00 mass% |
| Urea | 0.65 mass% |
| Dicalcium phosphate | 0.60 mass% |
| Calcium carbonate | 0.30 mass% |
| Dietary salt | 0.30 mass% |
| Vitamins A and D₂ premix | 0.07 mass% |
| Vitamin E premix | 0.05 mass% |
| Trace mineral premix | 0.04 mass% |
| Heated CNSL | 0.50 mass% |

(iv) Production Example 9: Production Example of feed for breeding young cows

| | |
|---|---|
| Standard feed for breeding young cows (SDC No. 2) (product of Nippon Formula Feed Mfg Co., Ltd.) | 99.50 mass% |
| Heated CNSL | 0.50 mass% |

### Industrial Applicability

According to the coccidiosis controlling agent of the present invention, and the additive and the feed of the present invention each containing the coccidiosis controlling agent, the coccidiosis of an animal (in particular, of livestock or pets) can be controlled.

## Claims

1. A coccidiosis controlling agent for an animal comprising heated cashew nut shell liquid and/or cardanol.

2. An additive for feed comprising the coccidiosis controlling agent according to claim 1.

3. A feed comprising the coccidiosis controlling agent according to claim 1 or the additive for feed according to claim 2.

4. A method for raising an animal comprising feeding the animal with the feed according to claim 3.

5. Heated cashew nut shell liquid and/or cardanol for use in producing a coccidiosis controlling agent for an animal.
